# EUROPEAN PATENT SPECIFICATION

(11) **EP 4 616 899 B1**
(45) Date of publication and mention of the grant of the patent: **07.10.2026**
(21) Application number: 25192971.7
(22) Date of filing: 16.02.2024
(51) Int. Cl.: A61N 1/05, A61N 1/362, A61N 1/372, A61N 1/375, A61N 1/39

(54) **PACING DEVICE HAVING PARTIALLY INSULATED TIP ELECTRODE**
SCHRITTMACHERVORRICHTUNG MIT TEILWEISE ISOLIERTER SPITZENELEKTRODE
DISPOSITIF DE STIMULATION CARDIAQUE A ELECTRODE A POINTE PARTIELLEMENT ISOLEE

(30) Priority: 17.02.2023 US 202363446779 P; 07.02.2024 US 202418435654
(43) Date of publication of application: 17.09.2025
(62) Document number(s) of the earlier application(s) in accordance with Art. 76 EPC: 24158012.5 / 4 417 246
(73) Proprietor: Pacesetter, Inc., Sylmar, CA 91342 (US)
(72) Inventor: FISHLER, Matthew G, Sylmar, CA 91342 (US); VICTORINE, Keith, Sylmar, CA 91342 (US); CHEN, Xiangqun Shawn, Sylmar, CA 91342 (US)
(74) Representative: Barker Brettell Sweden AB

(56) References cited:
- WO-A1-00/57949
- US-A1- 2010 292 768
- US-A1- 2020 289 835
- US-B1- 7 212 870

## Description

### TECHNICAL FIELD

The present disclosure relates to pacing devices. More specifically, the present disclosure relates to pacing leads and leadless biostimulators having tissue-piercing electrodes.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Cardiac pacing by currently available or conventional pacemakers is usually performed by a pulse generator implanted subcutaneously or sub-muscularly in or near a patient's pectoral region. Pulse generator parameters are usually interrogated and modified by a programming device outside the body, via a loosely coupled transformer with one inductance within the body and another outside, or via electromagnetic radiation with one antenna within the body and another outside. The pulse generator usually connects to the proximal end of one or more implanted leads, the distal end of which contains one or more electrodes for positioning adjacent to the inside or outside wall of a cardiac chamber. The leads have an electrical conductor for connecting the pulse generator to electrodes in the heart. Such electrode leads typically have lengths of 50 to 70 centimeters. Pacing leads can engage and/or be fixed to an intracardial implant site by an engaging mechanism such as an electrode anchor. For example, the electrode anchor can screw into the myocardium.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in or on a right ventricle and, for dual-chamber pacing, in a right atrium. Anchoring can occur through an anchor electrode that screws into the myocardium.

US 7,212,870 B1 discloses an implantable lead for use with an implantable medical device. The implantable lead includes a lead body with first and second electrical conductors extending between its proximal and distal ends. An electrical connector at the proximal end of the lead body includes terminals electrically connected to the first and second conductors. First and second coaxial active fixation helices are coupled to the lead body's distal end, one being an anode, the other an electrically isolated cathode. Each helix has an outer peripheral surface with alternating insulated and un-insulated portions along its length with about a half of the surface area being insulated. The un-insulated portions of the helices may be formed as a plurality of islands in the insulated portions, or as rings spaced by insulative rings, or as longitudinally extending strips spaced by longitudinally extending insulative strips.

US 2010/292768 A1 discloses electrical medical leads having active a fixation helix having selectively applied insulation to optimize exposed electrode surface area and dispose the exposed electrode surface area toward tissue that is less traumatized by injury caused by screwing in the fixation helix. An outer helical surface is masked by contact with a masking tube while a dielectric coating is applied to the inner helical surface of the coil turns of the helix, and the masking tube is removed when the dielectric coating has set. In one variation, at least one aperture is formed through the masking tube sidewall exposing an area of the outer helical surface thereby interrupting the uninsulated outer helical electrode.

US 2020/289835 A1 discloses a biostimulator, such as a leadless cardiac pacemaker, including coaxial fixation elements to engage or electrically stimulate tissue, is described. The coaxial fixation elements include an outer fixation element extending along a longitudinal axis and an inner fixation element radially inward from the outer fixation element. One or more of the fixation elements are helical fixation elements that can be screwed into tissue. The outer fixation element has a distal tip that is distal to a distal tip of the inner fixation element, and an axial stiffness of the outer fixation element is lower than an axial stiffness of the inner fixation element. The relative stiffnesses are based on one or more of material or geometric characteristics of the respective fixation elements. Other embodiments are also described and claimed.

### SUMMARY

The invention is defined in the independent claim. Further embodiments of the invention are defined in the dependent claims. Further aspects, embodiments and examples described hereinafter are only of exemplary nature and are presented for better understanding the present invention.

The disclosure relates to a pacing device, such as a pacing lead or biostimulator that includes a partially insulated tip electrode to control overall impedance of the pacing device.

According to the disclosure, the pacing device includes an electrical conductor having a longitudinal axis. The pacing device includes a tip electrode electrically coupled to the electrical conductor. The tip electrode extends along a spiral axis about the longitudinal axis. The tip electrode includes an outer radial surface and an inner radial surface. The tip electrode has a directionally-specific partial insulation including an insulative portion on the outer radial surface and a conductive portion along the inner radial surface resulting in an effective impedance based on an exposed surface area of the conductive portion on the inner radial surface. The effective impedance is higher than a comparative effective impedance based on the exposed surface area being on the outer radial surface.

### BRIEF DESCRIPTION OF THE DRAWINGS

The novel features of the invention are set forth with particularity in the claims that follow. A better understanding of the features and advantages of the present disclosure will be obtained by reference to the following detailed description that sets forth illustrative embodiments, in which the principles of the disclosure are utilized, and the accompanying drawings of which:
FIG. 1 is a diagrammatic medial-lateral cross section of a patient heart illustrating an example implantation of pacing devices in the patient heart, in accordance with an embodiment.
FIG. 2 is a side view of a pacing lead, in accordance with an embodiment.
FIG. 3 is a side view of a leadless biostimulator, in accordance with an embodiment.
FIG. 4 is a perspective view of a distal portion of a leadless biostimulator having a tip electrode and an outer fixation element, in accordance with an embodiment.
FIG. 5 is a cross-sectional view of a distal portion of a leadless biostimulator having a tip electrode and an outer fixation element, in accordance with an embodiment.
FIG. 6 is a partial side view of a distal portion of a pacing device, in accordance with an embodiment.
FIG. 7 is a cross-sectional view, taken about line A-A of FIG. 6, of a tip electrode, in accordance with an embodiment.
FIG. 8 is a partial side view of a distal portion of a pacing device, in accordance with an embodiment.
FIG. 9 is a partial side view of a distal portion of a pacing device, in accordance with an embodiment.
FIG. 10 is a graph of an impedance based on exposed angle of a tip electrode, in accordance with an embodiment.
FIG. 11 is a graph of an impedance based on exposed area of a tip electrode, in accordance with an embodiment.
FIG. 12 is a flowchart of a method of manufacturing a partially insulated tip electrode.
FIG. 13 is a perspective view of a mask disposed within an inner lumen of a tip electrode, in accordance with an embodiment.
FIG. 14 is a perspective view of a mask disposed over an outer radial surface of a tip electrode, in accordance with an embodiment.

### DETAILED DESCRIPTION

Embodiments describe a pacing device, e.g., a pacing lead or a leadless biostimulator, having a partially insulated tip electrode. The pacing device may be used to pace cardiac tissue as described below. The pacing device may, however, be used in other applications, such as deep brain stimulation. Thus, reference to the pacing device as being a cardiac pacing lead, biostimulator, or pacemaker is not limiting. Furthermore, the partially insulated tip electrode may be used in other active implantable medical device, such as a defibrillator, neurostimulator, etc. By way of example, active implantable medical devices having active fixation leads, such as implantable cardioverter-defibrillators, can incorporate the partially insulated tip electrode within the leads.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a pacing device. Similarly, "proximal" may indicate a second direction opposite to the first direction. Such terms are provided to establish relative frames of reference, however, and are not intended to limit the use or orientation of a pacing device to a specific configuration described in the various embodiments below.

Active implantable medical devices, such as pacing leads and leadless cardiac pacemakers, deliver output pulses to tissue via a set of electrodes, including an anchor electrode. An overall impedance for the output pulses is based on several factors, including: resistance associated with a lead or other internal components, characteristics of the electrodes and associated electrode-to-tissue interfaces, and conductivity of tissue between electrodes. The overall impedance directly impacts the efficiency and efficacy of tissue stimulation by the implantable medical device. Reducing the overall impedance can require more electrical current to drive pacing, thereby draining a battery faster and reducing device longevity. Conversely, increasing overall impedance can improve device longevity.

Overall impedance of existing stimulation electrodes is controlled by exposing a given amount of conductive surface area. For example, reducing exposed surface area of the electrode can increase overall impedance. Exposed surface area, however, is typically circumferentially around an entire electrode body, and thus, does not take advantage of relative impedance increases that can occur from selectively exposing directionally-specific surfaces of the electrode that have greater or lesser relative impacts on overall impedance. By contrast, the tip electrode described below selectively exposes specific surfaces of a tip electrode, e.g., inner or outer radial surfaces of the tip electrode, which can dramatically and/or asymmetrically increase an effective impedance of the tip electrode compared to exposure of the entire electrode body of the same tip electrode, or compared to selective symmetric exposure.

In an aspect, a pacing device is provided. The pacing device includes a partially insulated tip electrode to engage and stimulate a target tissue within a patient anatomy. In an embodiment, at a position along the tip electrode, a surface of the tip electrode has both an insulative portion and a conductive portion. An electric field of the tip electrode can therefore be modified at the position in such a way that an overall impedance of the tip electrode can be controlled. For example, the conductive portion may be along an inner radial surface of the tip electrode and at least a portion of the insulative portion may be along an outer radial surface of the tip electrode, at the position. Such directionally-specific partial insulation can result in an increase in overall impedance, as compared to a directionally-nonspecific partial insulation of the tip electrode. The directionally-specific partial insulation can also result in an increase in overall impedance, as compared to a shortened tip electrode having an equivalent amount of surface area as the tip electrode with directionally-specific partial insulation. The resulting increase in overall impedance can facilitate effective pacing of the target tissue with lower current draw from a battery.

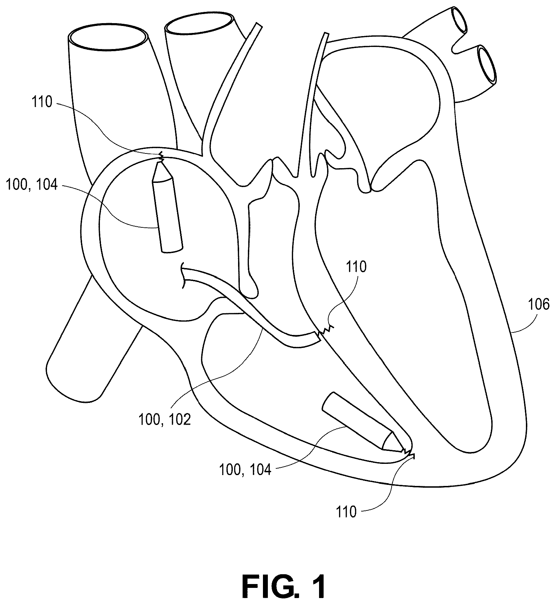

Referring to FIG. 1 a diagrammatic medial-lateral cross section of a patient heart illustrating an example implantation of pacing devices in the patient heart is shown in accordance with an embodiment. A biostimulator system, e.g., a cardiac pacing system, includes one or more pacing devices 100. The pacing devices 100 may include, for example, a pacing lead 102 or a leadless biostimulator 104. The pacing devices 100 can be implanted in the patient heart 106. Thus, the pacing devices 100 may be cardiac pacing leads or leadless cardiac pacemakers.

Each pacing device 100 can be placed in a cardiac chamber, such as a right atrium and/or right ventricle of the patient heart 106, or attached to an inside or outside of the cardiac chamber. Attachment of the pacing device 100 to the cardiac tissue can be accomplished via one or more fixation elements, such as the coaxial fixation elements described below. In a particular embodiment, the pacing device 100 includes a tip electrode 110 having a spiral, e.g., a helical configuration, to anchor in the cardiac tissue. The tip electrode 110 can also be an active electrode to pace the cardiac chamber upon receiving a triggering signal from circuitry within the biostimulator 104 or at least one other device within the body.

The pacing device 100 can be delivered to or retrieved from a patient using delivery or retrieval systems. The biostimulator system can include delivery or retrieval systems, which may be catheter-based systems used to carry a pacing lead 102 or a leadless biostimulator 104 intravenously to or from a patient anatomy. The delivery or retrieval systems may be referred to collectively as transport systems. In some implementations of transport systems, a leadless biostimulator 104 is attached or connected to a distal end of a catheter and advanced intravenously into or out of the heart 106. The transport system can include features to engage the leadless biostimulator 104 to enable fixation of the leadless biostimulator to tissue, and disengagement of the leadless biostimulator from tissue. For example, in implementations where the leadless biostimulator 104 includes a fixation mechanism, such as the tip electrode 110 or the outer fixation element described below, the transport system can include a docking cap or key at a distal end of the catheter, and the docking cap or key may be configured to engage the pacing device and apply torque to screw the fixation mechanism of the biostimulator into or out of the tissue. In other implementations, the transport system includes clips designed to match the shape of a feature on the pacing device and apply torque to screw the fixation mechanism of the biostimulator 104 into or out of the tissue.

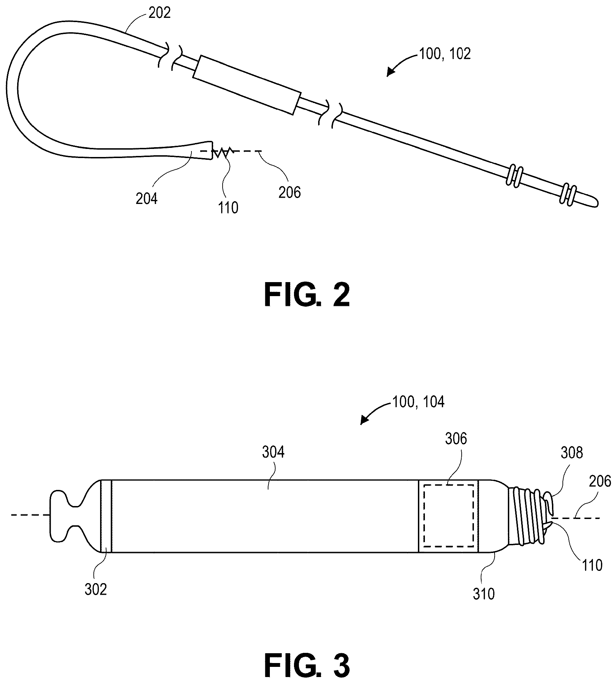

Referring to FIG. 2, a side view of a pacing lead is shown in accordance with an embodiment. The pacing device 100 can include a pacing lead 102 having an elongated lead body 202. The elongated lead body 202 can contain an electrical conductor 204, e.g., elongated electrical wires that are covered by an insulation sheath. The electrical conductor 204 can have a longitudinal axis 206. In an embodiment, the pacing lead 102 is flexible to allow intravenous insertion and delivery, and thus, the longitudinal axis 206 may be a central axis that is not always straight but conforms to the curve of the flexible lead.

In an embodiment, the pacing device 100 includes the tip electrode 110 electrically coupled to the electrical conductor 204 to receive and convey pacing impulses to the target tissue. The tip electrode 110 can extend along the longitudinal axis 206. For example, as described below, the tip electrode 110 can include a spiral or helical electrode extending along a spiral axis about the longitudinal axis 206. The tip electrode 110 may therefore anchor within and stimulate the target tissue.

Referring to FIG. 3, a side view of a leadless biostimulator is shown in accordance with an embodiment. The pacing device 100 can include a leadless biostimulator 104, e.g., a leadless cardiac pacemaker, which can perform cardiac pacing and that has many of the advantages of conventional cardiac pacemakers while extending performance, functionality, and operating characteristics. The biostimulator 104 can have two or more electrodes, e.g., the tip electrode 110 and a proximal electrode 302, located within, on, or near a housing 304 of the biostimulator 104. In an embodiment, the tip electrode 110 is a fixation element to anchor the biostimulator 104 in the target tissue. The electrodes can deliver pacing pulses to muscle of the cardiac chamber, and optionally, can sense electrical activity from the muscle. The electrodes may also communicate bidirectionally with at least one other device within or outside the body.

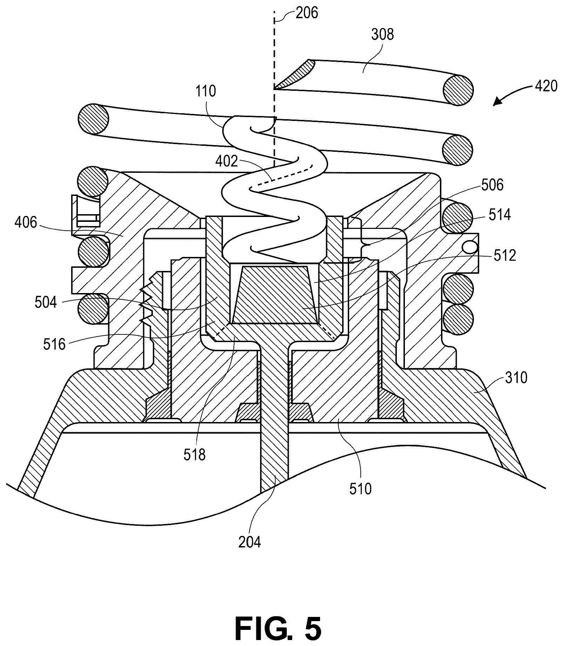

In an embodiment, the tip electrode 110 is mounted on the housing 304 along the longitudinal axis 206. As described above, the longitudinal axis 206 can be an axis of the electrical conductor 204 (not shown), which may include an electrode cup or electrode pin (FIG. 5), as described below. The housing 304 can contain a primary battery to provide power for pacing, sensing, and communication, which may include, for example, bidirectional communication. The housing 304 can optionally contain an electronics compartment 306 (shown by hidden lines) to hold circuitry adapted for different functionality. For example, the electronics compartment 306 can contain circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 104 can control these operations in a predetermined manner. In some implementations of a cardiac pacing system, cardiac pacing is provided without a pulse generator located in the pectoral region or abdomen, without an electrode-lead separate from the pulse generator, optionally without a communication coil or antenna, and optionally without an additional requirement of battery power for transmitting communication through a communication coil or antenna.

Leadless pacemakers or other leadless biostimulators can be fixed to an intracardial implant site by one or more actively engaging mechanism or fixation mechanism, such as a screw or helical member that screws into the myocardium. In an embodiment, the biostimulator 104 includes an outer fixation element 308 coupled to the housing 304. The outer fixation element 308 can be a helical element to screw into target tissue. More particularly, the outer fixation element 308 can extend helically from a flange 310 of the biostimulator 104, which is mounted on and can form a portion of the housing 304, to a distal tip of the helix. Accordingly, when the biostimulator 104 contacts the target tissue, the distal tip can pierce the tissue and the housing 304 can be rotated to screw the outer fixation element 308 into the target tissue to pull the tip electrode 110 into contact with the tissue. Rotation of the housing 304 can drive the outer fixation element 308 and the tip electrode 110 into the target tissue to anchor the biostimulator 104.

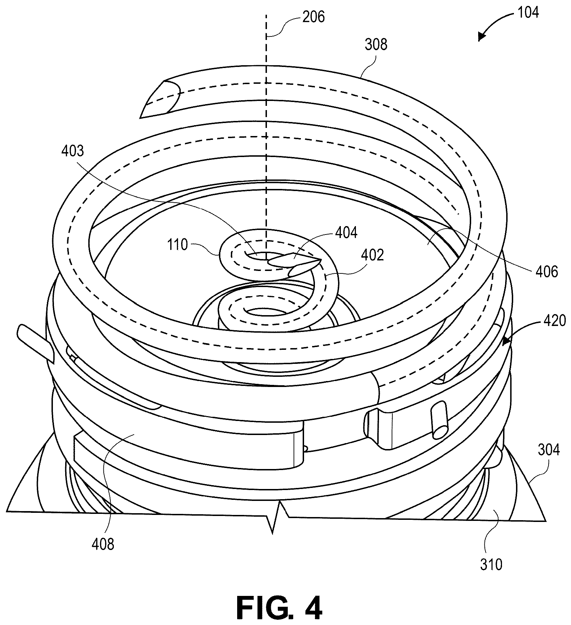

Referring to FIG. 4, a perspective view of a distal portion of a leadless biostimulator having a tip electrode and an outer fixation element is shown in accordance with an embodiment. The biostimulator 104 can include the tip electrode 110 coupled to the housing 304, e.g., via the flange 310. The tip electrode 110 can be coaxially arranged with the outer fixation element 308 about the longitudinal axis 206. More particularly, the tip electrode 110 can extend helically about the longitudinal axis 206 at a location that is radially inward from the outer fixation element 308. The outer fixation element 308 may therefore be coupled to the housing 304 radially outward of the tip electrode 110.

The tip electrode 110 can extend along a spiral axis 402 about the longitudinal axis 206. The helical configuration of the tip electrode 110 can define an inner lumen 403. More particularly, an inner lumen 403 can pass through the tip electrode 110 along the longitudinal axis 206. The inner lumen 403 is a space surrounded by the helical electrode, which may be exposed to a surrounding environment laterally through gaps between turns of the spiraling tip electrode 110.

The tip electrode 110 can extend distally to an electrode tip 404. The electrode tip 404 may be longitudinally proximal to, distal to, or coincident with a distal tip of the outer fixation element 308. The tip electrode 110 can be a helical element to screw into target tissue. Accordingly, when the outer fixation element 308 screws into the tissue, the electrode tip 404 can also engage the target tissue, and the housing 304 can be rotated to cause the electrode tip 404 of the tip electrode 110 to pierce the tissue and anchor the biostimulator 104.

The tip electrode 110 can be an active electrode, and can electrically communicate with the pacing circuitry contained in the electronics compartment 306. Accordingly, the anchored tip electrode 110 can electrically communicate with the tissue, and can transmit electrical pulses between the tissue and the pacing circuitry of the biostimulator 104. To facilitate electrical function of the tip electrode 110, and as described further below, the element may be coated in titanium nitride, iridium oxide, platinum black, etc. to reduce a polarization value of the tip electrode 110. By way of example, the titanium nitride coating may be in a range of 5 to 50 microns, e.g., 13 microns. The polarization value of the tip electrode 110 may similarly be reduced by etching a surface of the tip electrode using chemical or laser treatment.

The biostimulator 104 can include a header assembly 420. The header assembly 420 includes the outer fixation element 308 mounted on a helix mount 406. The helix mount 406 includes features that interact with the housing 304 and/or flange 310, the outer fixation element 308, and the target tissue. In an embodiment, the helix mount 406 includes a helix mount flange 408. The helix mount flange 408 can act as a mounting thread that extends around the longitudinal axis 206. A mounting thread structure of the helix mount flange 408 can be helical such that spiraling turns of the helix mount flange 408 are separated by helix mount gaps that also spiral about the longitudinal axis 206. The outer fixation element 308 can be received within the threaded groove (the helix mount gaps) of helix mount 406 as shown. For example, an assembler can insert a proximal end of the outer fixation element 308 into a distal end of the helical gap and rotate the outer fixation element 308 relative to the helix mount 406 to cause the helical fixation element to advance along and thread onto the helix mount 406.

Referring to FIG. 5, a cross-sectional view of a distal portion of a leadless biostimulator having a tip electrode and an outer fixation element is shown in accordance with an embodiment. In addition to the helix mount 406 and the outer fixation element 308, the header assembly 420 can include a flange 310 and the electrical conductor 204. The flange 310 can be an intermediate structure coupling the helix mount 406 to the housing 304. The electrical conductor 204, as described above, can interconnect the pacing circuitry to the tip electrode 110. For example, the electrical conductor 204 can be electrically coupled to the pacing circuitry, and can have an electrode pin extending along the longitudinal axis 206, and an electrode cup 504 disposed along the longitudinal axis 206. The electrode cup 504 can be mounted within the helix mount 406. For example, the electrode cup 504 can be radially inward of the helix mount flange 408, and can be longitudinally aligned with a distal opening of the helix mount 406. The header assembly 420 can also include an insulator 510 physically separating and electrically isolating the helix mount flange 408 from the electrode cup 504.

The tip electrode 110 can be electrically coupled to the electrical conductor 204 to receive pacing impulses from the pacing circuitry during operation. For example, the tip electrode 110 can be at least partially mounted in the electrode cup 504, in contact with a cup wall of the electrode cup 504. More particularly, a proximal portion 506 of the tip electrode 110 can be mounted in the electrode cup 504.

The tip electrode 110 can be connected to the electrode cup 504 in any of various manners, such as by thermally or adhesively welding the tip electrode 110 to the electrode cup 504. In an embodiment, the tip electrode 110 is attached to the electrode cup 504 by a weld, e.g., a spot weld. The weld can join the electrode cup 504 to the tip electrode 110 to cause the components to act as a unitary body under mechanical fatigue, e.g., when the tip electrode 110 is engaged in pulsating tissue. The weld can secure the tip electrode 110 relative to the electrode cup 504 in a particular orientation. Both the tip electrode 110 and the electrode cup 504 can be conductive, and thus, the unitary body provides an electrical pathway between the pacing circuitry and the target tissue.

The helix mount 406 can be mounted on the housing 304. For example, the helix mount 406 can have an internal thread that mounts on an external thread of the flange 310 by a threaded connection. Alternatively, an inner surface of the helix mount 406 can be cylindrically shaped and similar to an outer surface of the flange 310 such that the helix mount 406 can be press fit onto the flange 310. The press fit can form a press fit connection between the helix mount 406 and the flange 310. Both connection types are illustrated in FIG. 5 for brevity. It will be appreciated, however, that the biostimulator 104 may have only one of the connection types circumferentially around the helix mount 406. In an embodiment in which the inner surface of the helix mount 406 is wider than the outer surface of the flange 310, a spot weld or heat stake may be used to bond the helix mount 406 to the flange 310. Similarly, the helix mount 406 and the flange 310 can be connected by ultrasonic staking or mechanical interlock features.

In an embodiment, the biostimulator 104, and more particularly the electrode cup 504, can contain a filler 512. The filler 512 can be referred to as a monolithic controlled release device (MCRD). The MCRD can include a therapeutic material and can be loaded into the electrode cup 504. The therapeutic agent can include a corticosteroid, such as dexamethasone sodium phosphate, dexamethasone acetate, etc. Furthermore, the therapeutic agent can be loaded in a polymer, e.g., a silicone matrix. Accordingly, the filler 512 can deliver a specified dose of a therapeutic agent, e.g., a corticosteroid, into the target tissue. The therapeutic agent can be effectively delivered into the tissue after the biostimulator 104 is implanted in a patient. Accordingly, inflammation or injury of the captured tissue may be reduced.

The filler 512 can be contained in an electrode cavity 514 of the electrode cup 504. For example, the electrode cup 504 can include an electrode wall 516 extending distally from an electrode base 518. The electrode wall 516 can extend around the electrode cavity 514 to surround the filler 512 and separate the filler 512 from the insulator 510 or the flange 310 that is radially between the electrode cup 504 and the flange 310. The electrode cavity 514 can be located on the longitudinal axis 206. The electrode pin of the electrode cup 504 can extend proximally from the electrode base 518 along the longitudinal axis 206 through the insulator 510. The electrode pin can receive pacing impulses from pacing circuitry within the electronics compartment 306 and transmit the electrical signals to the tip electrode 110, which can act as a portion of the distal electrode to deliver the pacing impulses to the target tissue.

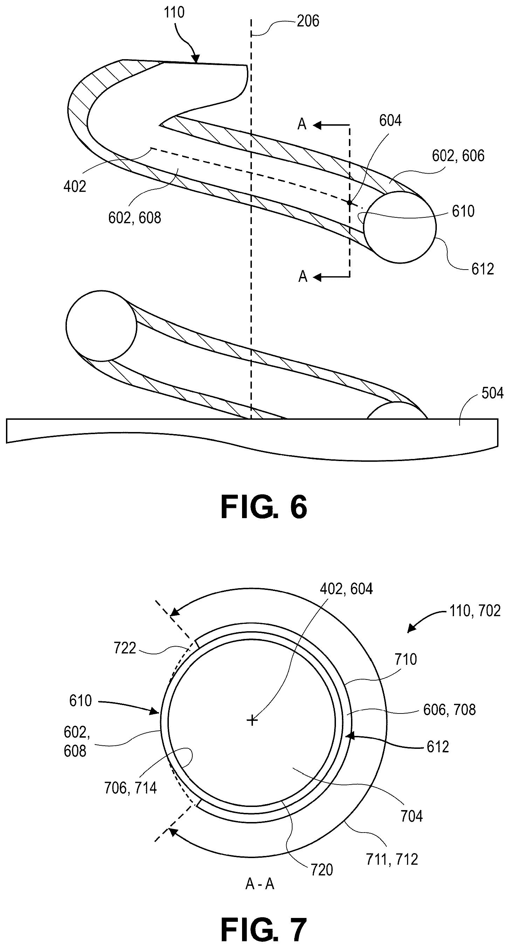

Referring to FIG. 6, a partial side view of a distal portion of a pacing device is shown in accordance with an embodiment. Having described an overall architecture of a pacing device having a tip electrode 110 coupled to an electrical conductor 204, both in a leaded and leadless platform, further description of the tip electrode 110 is now provided. A total exposed surface area of the tip electrode 110 may be controlled to provide a predetermined effective impedance of the electrode. For example, regardless of total surface area of the tip electrode 110, at least a portion of the tip electrode 110 can be insulated such that a total exposed surface area may be less than the total surface area of the tip electrode 110. By way of example, the total surface area of the tip electrode 110 may be 5 mm² and, in an embodiment, the tip electrode 110 can be partially insulated to reduce the exposed surface area to 2 mm², thereby increasing an effective impedance of the electrode. Accordingly, more than 25% of the total surface area, for example, in a range of 50 to 90%, e.g., 60%, of the total surface area can be insulated to increase the effective impedance of the electrode.

In an embodiment, the total exposed surface area is controlled to obtain a predetermined exposed area. For example, regardless of a total surface area of the tip electrode 110, the surface of the tip electrode can be partially insulated to provide an exposed area in a range of 0.5 to 4 mm². More particularly, the exposed area may be in a range of 1 to 2 mm², e.g., 1.5 mm². Targeting a final exposed surface area, rather than a percentage of insulated surface area, may provide a tip electrode 110 having favorable effective impedance over a range of electrode sizes, e.g., tip electrodes having a range of total surface areas.

The insulation process is described further below, and can include adding insulation material, e.g., parylene, to a surface 602 of the tip electrode 110. More particularly, the insulation material can be located on the surface 602 of the tip electrode 110 at a position 604 along the spiral axis 402. Accordingly, at the position 604, the tip electrode 110 has an insulative portion 606 and a conductive portion 608. The insulative portion 606 of the surface 602 includes the insulation material facing away from the longitudinal axis 206 toward the surrounding environment, and the conductive portion 608 of the surface 602 has an exposed surface area facing toward the longitudinal axis.

As described below, a position of the exposed surface area around the surface 602 of the tip electrode 110 can influence the effective impedance of the tip electrode 110, and thus, the position of the insulative portion 606 can be controlled. In particular, it has been shown that maintaining exposure of the conductive portion 608 of the tip electrode 110 along the inner radial surface 610 (the surface facing radially inward toward the longitudinal axis 206) of the tip electrode 110 can provide higher effective impedance than exposure of an equivalent surface area along an outer radial surface 612 (the surface facing radially outward away from the longitudinal axis 206). Furthermore, effective impedance may be influenced by exposing a distal portion, or a proximal portion, of the tip electrode 110. More particularly, effective impedance may be varied by insulating at least a portion of the tip electrode 110, and a degree of increase in the effective impedance can be controlled by insulating particular portions of the tip electrode 110 and/or by controlling a proportion of exposed area to insulated area at positions along the tip electrode 110.

In an embodiment, the conductive portion 608 of the tip electrode 110 spirals about the longitudinal axis 206. For example, the conductive portion 608 can include a conductive strip spiraling about the longitudinal axis 206 along the tip electrode 110. The conductive strip can extend along the inner radial surface 610 of the tip electrode 110. More particularly, the exposed surface area of the conductive portion 608 may be located radially inward of the spiral axis 402, e.g., between the spiral axis 402 and the longitudinal axis 206. The exposed surface area of the conductive portion 608 may therefore face toward the longitudinal axis 206.

As shown in FIG. 6, in contrast to the conductive portion, the insulative portion 606 of the tip electrode may include an insulative strip spiraling about the longitudinal axis along the tip electrode 110 at locations diametrically opposed to the exposed area. For example, at least a portion of the insulative strip may extend along the outer radial surface 612 of the tip electrode 110. The insulative strip may therefore face away from the longitudinal axis 206. The insulative portion 606 may be above, below, or radially outward of the spiral axis 402, or combinations thereof.

Referring to FIG. 7, a cross-sectional view, taken about line A-A of FIG. 6, of a tip electrode is shown in accordance with an embodiment. A cross-section 702 of the tip electrode 110 taken transverse to the spiral axis 402 at the position 604 reveals several components of the electrode forming the conductive portion 608 and the insulative portion 606. The tip electrode 110 includes a core wire 704 that is conductive and underlies both the conductive portion 608 and the insulative portion 606. For example, the core wire 704 can include a platinum iridium (Pt 90/Ir 10) wire formed into the spiral shape. The core wire 704 has a perimeter 706, and a surface of the core wire 704 along the perimeter 706 faces radially outward from the spiral axis 402.

In an embodiment, the insulative portion 606 includes an insulative layer 708 extending over a portion of the perimeter 706. The insulative layer 708 can extend over an arc 710. The arc 710 is a curve that does not extend over an entire circle. More particularly, the arc 710 can have an arc angle 711 that is less than 360 degrees. As such, the arc 710 can extend around the perimeter 706, however, the arc 710 may have an arc length 712 that is less than a perimeter length 714 (e.g., a circumference) of the perimeter 706. Accordingly, at least a portion of the tip electrode 110 is exposed conductive area facing outward and surrounded by the insulative portion 606.

An effective impedance of the tip electrode 110 is influenced by an amount of exposed area. The arc length 712 of the insulative layer 708 can therefore be varied to achieve a predetermined effective impedance. The exposed surface area can cover 240 degrees or less of the tip electrode circumference. In an embodiment, the arc length 712 of the insulative layer 708 is at least half of the perimeter length 714. Accordingly, less than half of a cross-sectional perimeter 706 of the tip electrode 110 at the position 604 may be exposed area. For example, an angle of exposure of the conductive portion 608 may be 180 degrees or less, e.g., 120 degrees. In an embodiment, the angle of exposure is less than 120 degrees, e.g., 90 degrees. Alternatively, the angle of exposure may be less than 90 degrees, e.g., 60 degrees. Reducing exposed surface area can increase effective impedance, however, the increase in impedance may be balanced against the need to capture and stimulate the target tissue. Accordingly, there may be a minimum exposed surface angle. For example, the angle of exposure may be 15 degrees or more.

The conductive portion 608 of the tip electrode 110 may include the core wire 704 with or without a surface modification 720 on the core wire 704. The surface modification 720 can extend over at least the conductive portion 608 of the tip electrode 110. For example, the tip electrode 110 can cover all or a portion of the core wire 704. The tip electrode 110 can include the surface modification 720 between the core wire 704 and the insulative portion 606. The surface modification 720 can be conductive. For example, the surface modification 720 can include a low-polarization coating on the core wire 704. The low-polarization coating can include titanium nitride or platinum black. The coating may be layered over the perimeter 706 of the core wire 704 in a continuous or patterned configuration. For example, the coating layer may cover an entire circumference of the core wire 704, or only a portion of the core wire 704, such as the portion underlying the conductive portion 608 of the surface 602 of the tip electrode 110.

The surface modification 720 may, rather than being a separate material from the core wire 704, include a modification of the core wire material. For example, the surface modification 720 can include laser restructuring of the core wire surface. The laser restructuring can impart electrical characteristics, such as a reduction in polarization of the surface. Accordingly, the conductive portion 608 of the tip electrode 110 can include the core wire 704 with or without additional conductive components that are in electrical contact with the core wire 704.

In an embodiment, the tip electrode 110 includes a transition zone 722 between the insulative portion 606 and the conductive portion 608. The transition zone 722 can be a region extending in a circumferential direction over which characteristics of the insulation varies relative to the insulative portion 606. For example, a coating density of the insulative portion 606 may be greater than a coating density of the transition zone 722. Stated differently, the coating density of the insulative portion 606 may decrease along the perimeter 706 over the transition zone 722. The decrease in coating density can be in a direction of the conductive portion 608. For example, a thickness of the insulation over the transition zone 722 may gradually decrease from a maximum thickness at the insulative portion 606 to a minimum thickness at the conductive portion 608. More particularly, the insulation thickness can taper from the maximum thickness to zero at the exposed surface area of the conductive portion 608.

The transition zone 722 having the insulation thickness variation can influence edge effects of the insulative portion 606. Insulating the surface 602 of the tip electrode 110 may produce a localized increase in current density at a location where the insulative portion 606 transitions to the conductive portion 608. Such increase in current density may result in suboptimal tissue stimulation. The transition zone 722, which can gradually blend the insulative portion 606 into the conductive portion 608 can reduce the localized current density at the transition, which may lead to comparatively advantageous tissue stimulation.

The transition zone 722, which transitions the insulative portion 606 into the conductive portion 608, may be formed in numerous manners. By way of example, the insulative portion 606 may be deposited on the core wire 704 and/or the surface modification 720 in a spraying process. An insulation material may be sprayed on to the underlying conductive wire structure. In an embodiment, the conductive portion 608 may be masked during the spraying process to prevent the sprayed insulation from covering the predetermined exposed surface area. Some overspray may occur at the mask, however, and a portion of the sprayed insulation may migrate below the mask toward the exposed area. The overspray region may be thinner than the main area sprayed over the insulative portion 606. Accordingly, a thickness transition can occur between the insulative portion 606 and the conductive portion 608.

Positioning of the conductive portion 608 at the inner radial surface 610 and at least a portion of the insulative portion 606 at the outer radial surface 612, as described above, is provided by way of example only. In at least one position 604 along the spiral axis 402, the insulative portion 606 may be located on the inner radial surface 610. For example, in the examples above, the positioning of the conductive portion 608 and the insulative portion 606 may be swapped. More particularly, the shaded area of the tip electrode 110 in FIG. 6 may be the conductive portion 608 and the unshaded area may be the insulative portion 606. Similarly, the locations indicated as the inner radial surface 610 and the outer radial surface 612 in FIG. 7 may be reversed.

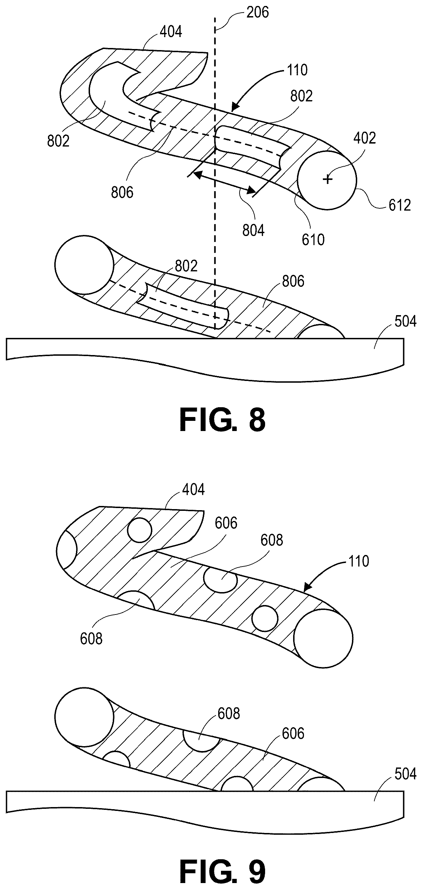

Referring to FIG. 8, a partial side view of a distal portion of a pacing device is shown in accordance with an embodiment. Similar to altering the radial position of the insulative portion 606, the insulative portion 606 and the conductive portion 608 may or may not extend continuously along the helical electrode. In an embodiment, the conductive portion 608 includes a conductive strip 802 spiraling about the longitudinal axis 206 along the tip electrode 110. The conductive strip 802 can be a continuous strip extending from the electrode cup 504 to the electrode tip 404. The conductive strip 802 may, for example, extend continuously along the inner radial surface 610 or the outer radial surface 612 of the tip electrode 110. The conductive strip 802 may therefore face toward or away from the longitudinal axis 206 over an entire length of the tip electrode 110 exposed distal to the electrode cup 504. The conductive strip 802 may, however, extend discontinuously along the tip electrode 110.

In an embodiment, the conducting strip has a strip length measured from a proximal edge of the strip, nearest to the electrode cup 504 in a direction of the spiral axis 402, to a distal edge of the strip, nearest to the electrode tip 404 in the direction of the spiral axis 402. The conductive strip 802 can extend along the spiral axis 402, and thus, the strip length may be a spiral length 804. The tip electrode 110 can include several turns about the longitudinal axis 206. The spiral length 804, however, may be less than a length of the several turns. For example, the spiral length 804 of the conductive strip 802 can be less than one turn of the several turns. The conductive strip 802 may therefore be discontinuous, providing for at least a portion of the insulative portion 606 to be exposed at a same radial location as the conductive strip 802, proximal and/or distal to the strip in the direction of the spiral axis 402.

The conductive strip 802 may extend along the tip electrode 110 at a same radial location on the tip electrode 110 relative to the spiral axis 402. For example, the conductive strip 802 shown in FIG. 8 extends along the radially inward surface 610 of the tip electrode 110 such that an axis drawn through the spiral axis 402 and orthogonal to the longitudinal axis 206 would intersect the conductive strip 802 at any position along the spiral axis 402 over the spiral length 804.

The conductive strip 802 may have a midline 806, which defines a radial location of the conductive strip 802 relative to the spiral axis 402. The midline 806 can be located at a point on the surface 602 of the tip electrode 110 that is a midpoint of an arc length of the conductive portion 608. More particularly, an area of the conductive portion 606 on a first side of the midline 806 in a circumferential direction along the surface 602 of the tip electrode 110 can have approximately half of the arc length, and another area of the conductive portion 608 on a second side of the midline 806 in the circumferential direction along the surface 602 of the tip electrode 110 can have approximately half of the arc length. The midline 806 can therefore bisect the arc of the conductive strip 802.

In an embodiment, the conductive strip 802 extends along the tip electrode 110 at different radial positions relative to the spiral axis 402. The midline 806 of the conductive strip 802 can extend along the surface 602 of the tip electrode 110 through locations that are at different radial locations relative to the strip axis. More particularly, a radial distance from the midline 806 of the conductive strip 802 to the longitudinal axis 206 can vary along the tip electrode 110. For example, the midline 806, and thus the conductive strip 802, can spiral around the tip electrode 110 in the direction of the spiral axis 402. The conductive strip 802 may therefore form a spiral about a spiral. The conductive strip 802 can be a single, continuous strip over the electrode length. Alternatively, the tip electrode 110 can have several, discontinuous strips of insulation separated from each other by intervening conductive areas.

In an embodiment, the insulative strip that, as described above, can be diametrically opposed to the conductive strip 802, may extend along the tip electrode 110 at different radial positions relative to the spiral axis 402. A midline of the insulative strip, which can be opposite of the midline 806 of the conductive strip 802, can extend along the surface 602 of the tip electrode 110 through locations that are at different radial locations relative to the strip axis. More particularly, a radial distance from the midline of the insulative strip to the longitudinal axis 206 can vary along the tip electrode 110. For example, the midline, and thus the insulative strip, can spiral around the tip electrode 110 in the direction of the spiral axis 402. The insulative strip may therefore form a spiral about a spiral.

Referring to FIG. 9, a partial side view of a distal portion of a pacing device is shown in accordance with an embodiment. The conductive strips 802 described above are illustrative, and not limiting, of the partially insulated electrode configurations. Other partial insulation patterns may be contemplated. For example, the conductive portion 608 can include several discrete regions of conduction disposed on the surface 602 of the tip electrode 110. The discrete regions can, for example, form a polka-dotted pattern on the surface. By way of example, the conductive portions 606 may be circular regions of conduction of the underlying conductive wire structure exposed through the insulation portion 606. The circular conductive regions can be exposed by removing, e.g., laser ablating, portions of a deposited insulation layer to selectively reveal the underlying conductive surface. The deposits can produce some cross-sections 702 of the tip electrode 110 that have fully insulated portions 606 around the perimeter 706. Adjacent cross-sections 702 may, however, include partially insulated perimeters 706, such as shown in FIG. 7. Accordingly, the embodiments described above will be understood to be illustrative, and not exhaustive, of the partially insulated tip electrode 110.

One or more turns may be entirely exposed or entirely insulated. For example, one or more proximal turns of the tip electrode 110 may be fully exposed. In addition to affecting effective impedance of the exposed turns, and the tip electrode 110 as a whole, the exposed surface area of the proximal turns can facilitate manufacturing. For example, the exposed proximal turns may be welded to the exposed internal surfaces of the cup walls of the electrode cup 504. Accordingly, surface area may be selectively insulated both to enhance impedance and to facilitate manufacturing of the pacing device 100.

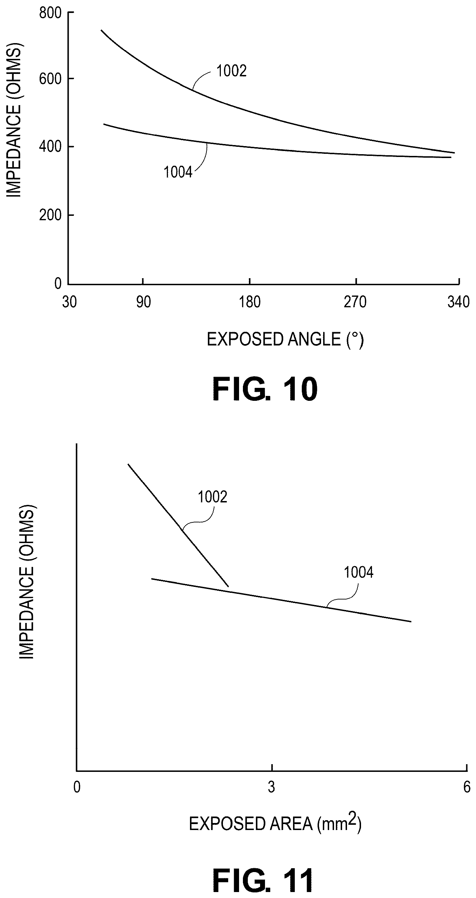

Referring to FIG. 10, a graph of an impedance based on exposed angle of a tip electrode is shown in accordance with an embodiment. An amount of exposed surface area can impact effective impedance of the tip electrode 110. For example, as shown in the graphs, increased exposed angle can reduce the effective impedance both for an inner surface exposure 1002 and an outer surface exposure 1004. The inner surface exposure 1002 refers to the exposed area of the tip electrode 110 facing toward the longitudinal axis 206, as shown in FIG. 7. By contrast, the outer surface exposure 1004 refers to the exposed area of the tip electrode 110 facing away from the longitudinal axis 206.

The curves illustrate that not only does exposed angle impact effective impedance, but the radial location of the exposed surface area can also impact effective impedance of the tip electrode 110. More particularly, it is evident that the inner surface exposure 1002 curve increases comparatively more, per decreased angle of exposure, than the outer surface exposure 1004 curve. Such distinction in the trend lines indicates that exposed angle on the inside surface of the tip electrode 110 has a higher impedance as compared to equivalent solid angle exposure along the outside surface of the tip electrode 110.

Referring to FIG. 11, a graph of an impedance based on exposed area of a tip electrode is shown in accordance with an embodiment. The trend in relative increase in impedance for inner surface exposure 1002 as compared to outer surface exposure 1004 also holds based on exposed surface area. More particularly, when plotting effective impedance against exposed surface area, the impedance trend increases comparatively more for the inner surface exposure 1002 curve than the outer surface exposure 1004 curve. Such trends indicate that not only are different, and higher, output impedances achieved for locating the conductive portion 608 along the inner radial surface 610 (as compared to locating the conductive portion 608 along the outer radial surface 612), but such comparative differences are present for the same exposed angle and the same coverage area. The tip electrode 110 may therefore have the insulative portion 606 along the outer radial surface 612 to provide higher impedance for a same arc angle 711 of the insulative portion 606.

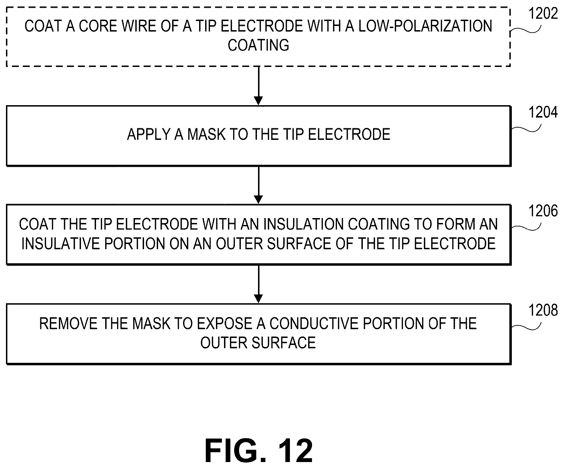

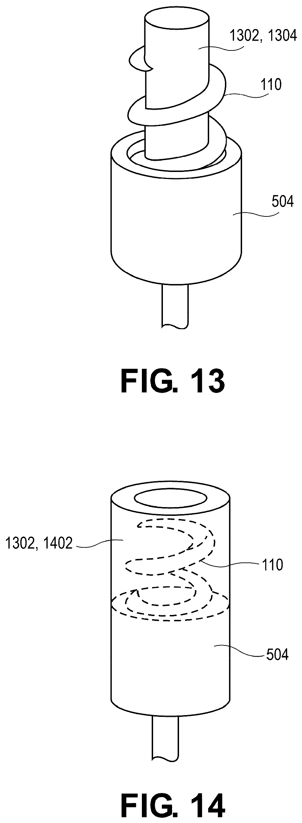

Referring to FIG. 12, a flowchart of a method of manufacturing a partially insulated tip electrode is shown in accordance with an embodiment. Operations of the method are illustrated in FIGS. 13-14, and thus, FIGS. 12-14 are referred to alternately in the description below.

Optionally, at operation 1202, the surface modification 720 may be disposed on the core wire 704, before or after turning the wire into a spiral structure. For example, the core wire 704 may be coated with the low-polarization coating. Alternatively, the outer surface of the core wire 704 can be surface modified, e.g., via laser restructuring, to produce a conductive wire structure having the core wire 704 and/or a conductive coating facing radially outward from the spiral axis 402.

Selective coating of the tip electrode 110 may be achieved by masking a selective area. At operation 1204, a mask is applied to the tip electrode 110. Various masking techniques may be used depending upon whether the exposed area is intended to be at the inner radial surface 610 or the outer radial surface 612. Several such masking techniques are described below.

Referring to FIG. 13, a perspective view of a mask disposed within an inner lumen of a tip electrode is shown in accordance with an embodiment. The method includes applying a mask 1302 to the tip electrode 110. Applying the mask 1302 can include disposing a plug 1304 within the inner lumen 403 of the tip electrode 110. The plug 1304 may have a cylindrical shape. For example, the plug 1304 can include a silicone rubber rod, which may be inserted into the inner lumen 403 of the tip electrode 110. The plug 1304 can have a diameter that is slightly larger than the inner diameter of the tip electrode 110. Accordingly, the plug 1304 can form a press fit against the inner radial surface 610 of the tip electrode 110. A size of the plug 1304 may be selected such that the plug 1304 compresses against and masks a region of the tip electrode 110 that will become the conductive portion 608 after a coating operation.

Referring to FIG. 14, a perspective view of a mask disposed over an outer radial surface of a tip electrode is shown in accordance with an embodiment. When the outer radial surface 612, rather than the inner radial surface 610, is intended to be exposed for pacing, applying the mask 1302 can include covering the outer radial surface 612 of the tip electrode 110 with the mask 1302. The mask 1302 can include a sleeve 1402, e.g., a tubular structure, which can be loaded over the tip electrode 110. The sleeve 1402 may include a silicone tubing, which may be inserted over, e.g., slid over, the outer radial surface 612 of the tip electrode 110. Alternatively, the sleeve 1402 may be formed from heat shrink tubing, which may be fit onto, e.g., shrunk over, the outer radial surface 612 of the tip electrode 110. The sleeve 1402 can have an inner diameter that is slightly smaller than the outer radial surface diameter of the tip electrode 110. Accordingly, the sleeve 1402 can form a press fit against the outer radial surface 612 of the tip electrode 110. A size of the sleeve 1402 may be selected such that the sleeve compresses against and masks a region of the tip electrode 110 that will become the conductive portion 608 after a coating operation.

At operation 1206, the tip electrode 110 can be coated with an insulation coating to form the insulative portion 606 on the surface 602 of the tip electrode 110. Various coating techniques may be used. For example, coating the tip electrode 110 can include depositing a conformal film over the core wire 704 of the tip electrode 110. The conformal film may be a parylene film deposited over the core wire 704 and/or the surface modification 720 in a chemical vapor deposition process. It will be appreciated that other coating techniques may be used. For example, an insulation material may be sprayed or painted onto the surface 602 of the conductive wire structure. Alternatively, the wire structure can be dipped in the insulation material. In any case, the surface 602 of the tip electrode 110, including the mask 1302, can be covered by the insulation material.

At operation 1208, the mask 1302 is removed. The mask 1302 may be stripped away from the tip electrode 110 using chemical or thermal removal processes. The mask 1302 may alternatively be mechanically removed, e.g., pulling the mask out or off, rotating the mask off, peeling the mask off, etc. Removal of the mask 1302 can expose the conductive portion 608 of the surface 602 of the tip electrode 110.

The method described above is an additive process in which the tip electrode 110 is masked and then insulated. In an embodiment, a reductive process may be used to manufacture the partially insulated tip electrode 110. For example, an entire surface of the tip electrode 110 may be insulated, e.g., by depositing parylene over the surface 602 of the conductive wire structure. The parylene may then be selectively removed, e.g., using an ablation laser, to locally expose surface area of the conductive portion 608. Such reductive processes may not require adding or removing the mask 1302.

The partially insulated tip electrode 110 may be manufactured at the component level using the method described above. Alternatively, the tip electrode 110 may be assembled to the electrode cup 504 and/or other components of the pacing device 100, and then partially insulated using the above method. In any case, the partially insulated tip electrode 110 may be integrated within the pacing device 100, e.g., as part of a biostimulator 104, to pace the target tissue.

In an embodiment, the coating on the tip electrode may include a therapeutic agent. For example, in addition to having an insulative material, the coating may also include the therapeutic agent to treat the target tissue. The therapeutic agent can be a drug, e.g., a biologically active compound, capable of treating a condition of the target tissue. For example, the therapeutic agent may be a steroid capable of treating inflammation of the target tissue. The steroid may be on or in the insulative portion 606, e.g., dispersed within the insulation coating. Accordingly, the steroid can be eluted into the target tissue from the coating when the tip electrode is implanted in the target tissue. The steroid can reduce inflammation of the target tissue.

## Claims

1. A pacing device (100), comprising:
an electrical conductor (204) having a longitudinal axis (206); and
a tip electrode (110) electrically coupled to the electrical conductor (204) and extending along a spiral axis (402) about the longitudinal axis (206), wherein the tip electrode (110) includes an outer radial surface (612) and an inner radial surface (610), wherein the tip electrode (100) has a directionally-specific partial insulation including an insulative portion (606) on the outer radial surface (612) and a conductive portion (608) along the inner radial surface (610) resulting in an effective impedance based on an exposed surface area of the conductive portion (608) on the inner radial surface (610), and wherein the effective impedance is higher than a comparative effective impedance based on the exposed surface area being on the outer radial surface (612).

2. The pacing device of claim 1, wherein the exposed surface area includes an exposed area of the tip electrode (110) facing the longitudinal axis (206).

3. The pacing device of claim 2, wherein a cross-section of the tip electrode (110) taken transverse to the spiral axis (402) at a position includes a core wire (704) having a perimeter (706), and wherein the insulative portion (606) includes an insulative layer (708) extending over an arc (710) around the perimeter (706).

4. The pacing device of claim 3, wherein the arc (710) has an arc length that is less than a perimeter length of the perimeter (706).

5. The pacing device of claim 4, wherein the arc length is at least half of the perimeter length.

6. The pacing device of any one of claims 1 to 5, wherein the insulative portion (606) includes an insulative strip spiraling about the longitudinal axis (206) along the tip electrode (110).

7. The pacing device of claim 6, wherein the outer radial surface (612) faces away from the longitudinal axis (206), and wherein at least a portion of the insulative strip extends along the outer radial surface (206).

8. The pacing device of any one of claims 1 to 7, wherein the effective impedance increases comparatively more, per decreased angle of the exposed surface area, than the comparative effective impedance.

9. The pacing device of any one of claims 1 to 8, wherein the exposed surface area is smaller than a covered surface area on the outer radial surface (612).

10. The pacing device of any one of claims 3 to 5, further comprising a surface modification extending over at least the exposed surface area of the tip electrode (110).

11. The pacing device of claim 10, wherein the surface modification includes a low-polarization coating on the core wire (704).

12. The pacing device of any one of claims 1 to 11, further comprising a housing (304) including an electronics compartment (306) containing pacing circuitry, wherein the electrical conductor (204) is electrically coupled to the pacing circuitry.

13. The pacing device of claim 12, further comprising an outer fixation element (308) coupled to the housing (304) radially outward of the tip electrode (110).

14. The pacing device of claim 13, further comprising a helix mount (406), wherein the outer fixation element (308) is mounted on the helix mount (406), and wherein the electrical conductor (204) extends through a cavity within the helix mount (406).

## Patentansprüche

1. Schrittmachervorrichtung (100), umfassend:
einen elektrischen Leiter (204), der eine Längsachse (206) aufweist; und
eine Spitzenelektrode (110), die elektrisch mit dem elektrischen Leiter (204) gekoppelt ist und sich entlang einer Spiralachse (402) um die Längsachse (206) erstreckt, wobei die Spitzenelektrode (110) eine äußere radiale Oberfläche (612) und eine innere radiale Oberfläche (610) beinhaltet, wobei die Spitzenelektrode (100) eine richtungsspezifische Teilisolierung aufweist, die einen isolierenden Abschnitt (606) auf der äußeren radialen Oberfläche (612) und einen leitfähigen Abschnitt (608) entlang der inneren radialen Oberfläche (610) beinhaltet, was zu einer effektiven Impedanz führt, basierend auf einem exponierten Oberflächenbereich des leitfähigen Abschnitts (608) auf der inneren radialen Oberfläche (610), und wobei die effektive Impedanz höher als eine vergleichende effektive Impedanz ist, basierend darauf, dass sich der exponierte Oberflächenbereich auf der äußeren radialen Oberfläche (612) befindet.

2. Schrittmachervorrichtung nach Anspruch 1, wobei der exponierte Oberflächenbereich einen exponierten Bereich der Spitzenelektrode (110) beinhaltet, welcher der Längsachse (206) zugewandt ist.

3. Schrittmachervorrichtung nach Anspruch 2, wobei ein Querschnitt der Spitzenelektrode (110), der an einer Position quer zu der Spiralachse (402) genommen wird, einen Kerndraht (704) beinhaltet, der einen Umfang (706) aufweist, und wobei der isolierende Abschnitt (606) eine isolierende Schicht (708) beinhaltet, die sich über einen Bogen (710) um den Umfang (706) erstreckt.

4. Schrittmachervorrichtung nach Anspruch 3, wobei der Bogen (710) eine Bogenlänge aufweist, die kleiner als eine Umfangslänge des Umfangs (706) ist.

5. Schrittmachervorrichtung nach Anspruch 4, wobei die Bogenlänge mindestens die Hälfte der Umfangslänge beträgt.

6. Schrittmachervorrichtung nach einem der Ansprüche 1 bis 5, wobei der isolierende Abschnitt (606) einen isolierenden Streifen beinhaltet, der entlang der Spitzenelektrode (110) spiralförmig um die Längsachse (206) verläuft.

7. Schrittmachervorrichtung nach Anspruch 6, wobei die äußere radiale Oberfläche (612) von der Längsachse (206) weg gewandt ist und wobei sich mindestens ein Abschnitt des isolierenden Streifens entlang der äußeren radialen Oberfläche (206) erstreckt.

8. Schrittmachervorrichtung nach einem der Ansprüche 1 bis 7, wobei die effektive Impedanz pro verringertem Winkel des exponierten Oberflächenbereichs vergleichsweise stärker zunimmt als die vergleichende effektive Impedanz.

9. Schrittmachervorrichtung nach einem der Ansprüche 1 bis 8, wobei der exponierte Oberflächenbereich kleiner als ein abgedeckter Oberflächenbereich auf der äußeren radialen Oberfläche (612) ist.

10. Schrittmachervorrichtung nach einem der Ansprüche 3 bis 5, ferner umfassend eine Oberflächenmodifikation, die sich über mindestens den exponierten Oberflächenbereich der Spitzenelektrode (110) erstreckt.

11. Schrittmachervorrichtung nach Anspruch 10, wobei die Oberflächenmodifikation eine Beschichtung mit niedriger Polarisation auf dem Kerndraht (704) beinhaltet.

12. Schrittmachervorrichtung nach einem der Ansprüche 1 bis 11, ferner umfassend ein Gehäuse (304), das ein Elektronikfach (306) beinhaltet, das eine Schrittmacherschaltung enthält, wobei der elektrische Leiter (204) elektrisch mit der Schrittmacherschaltung gekoppelt ist.

13. Schrittmachervorrichtung nach Anspruch 12, ferner umfassend ein äußeres Fixierungselement (308), das radial auswärts der Spitzenelektrode (110) an das Gehäuse (304) gekoppelt ist.

14. Schrittmachervorrichtung nach Anspruch 13, ferner umfassend eine Schrägungsaufnahme (406), wobei das äußere Fixierungselement (308) auf der Schrägungsaufnahme (406) montiert ist und wobei sich der elektrische Leiter (204) durch einen Hohlraum innerhalb der Schrägungsaufnahme (406) erstreckt.

## Revendications

1. Dispositif de stimulation cardiaque (100), comprenant :
un conducteur électrique (204) présentant un axe longitudinal (206) ; et
une électrode à pointe (110) reliée électriquement au conducteur électrique (204) et s'étendant le long d'un axe en spirale (402) autour de l'axe longitudinal (206), dans lequel l'électrode à pointe (110) comprend une surface radiale externe (612) et une surface radiale interne (610), dans lequel l'électrode à pointe (100) présente une isolation partielle à spécificité directionnelle incluant une partie isolante (606) sur la surface radiale externe (612) et une partie conductrice (608) le long de la surface radiale interne (610) se traduisant par une impédance effective basée sur une surface exposée de la partie conductrice (608) sur la surface radiale interne (610), et dans lequel l'impédance effective est supérieure à une impédance effective comparative basée sur une configuration dans laquelle la surface exposée se trouve sur la surface radiale externe (612).

2. Dispositif de stimulation cardiaque selon la revendication 1, dans lequel la surface exposée comprend une zone exposée de l'électrode à pointe (110) tournée vers l'axe longitudinal (206).

3. Dispositif de stimulation cardiaque selon la revendication 2, dans lequel une section transversale de l'électrode à pointe (110), prise transversalement à l'axe en spirale (402) en une position donnée, comprend un fil d'âme (704) présentant un périmètre (706), et dans lequel la partie isolante (606) comprend une couche isolante (708) s'étendant sur un arc (710) autour du périmètre (706).

4. Dispositif de stimulation cardiaque selon la revendication 3, dans lequel l'arc (710) présente une longueur d'arc qui est inférieure à une longueur circonférentielle du périmètre (706).

5. Dispositif de stimulation cardiaque selon la revendication 4, dans lequel la longueur d'arc représente au moins la moitié de la longueur circonférentielle.

6. Dispositif de stimulation cardiaque selon l'une quelconque des revendications 1 à 5, dans lequel la partie isolante (606) comprend une bande isolante s'enroulant en spirale autour de l'axe longitudinal (206) le long de l'électrode à pointe (110).

7. Dispositif de stimulation cardiaque selon la revendication 6, dans lequel la surface radiale externe (612) est tournée à l'opposé de l'axe longitudinal (206), et dans lequel au moins une partie de la bande isolante s'étend le long de la surface radiale externe (206).

8. Dispositif de stimulation cardiaque selon l'une quelconque des revendications 1 à 7, dans lequel l'impédance effective augmente comparativement davantage, pour chaque diminution de l'angle de la surface exposée, que l'impédance effective comparative.

9. Dispositif de stimulation cardiaque selon l'une quelconque des revendications 1 à 8, dans lequel la surface exposée est inférieure à une surface recouverte sur la surface radiale externe (612).

10. Dispositif de stimulation cardiaque selon l'une quelconque des revendications 3 à 5, comprenant en outre une modification de surface s'étendant sur au moins la surface exposée de l'électrode à pointe (110).

11. Dispositif de stimulation cardiaque selon la revendication 10, dans lequel la modification de surface comprend un revêtement à faible polarisation sur le fil d'âme (704).

12. Dispositif de stimulation cardiaque selon l'une quelconque des revendications 1 à 11, comprenant en outre un boîtier (304) incluant un compartiment électronique (306) contenant des circuits de stimulation, dans lequel le conducteur électrique (204) est relié électriquement aux circuits de stimulation.

13. Dispositif de stimulation cardiaque selon la revendication 12, comprenant en outre un élément de fixation externe (308) relié au boîtier (304) radialement vers l'extérieur par rapport à l'électrode à pointe (110).

14. Dispositif de stimulation cardiaque selon la revendication 13, comprenant en outre un support d'hélice (406), dans lequel l'élément de fixation externe (308) est monté sur le support d'hélice (406), et dans lequel le conducteur électrique (204) s'étend à travers une cavité à l'intérieur du support d'hélice (406).
